# EUROPÄISCHE PATENTSCHRIFT

(11) **EP 3 442 354 B1**
(45) Veröffentlichungstag und Bekanntmachung des Hinweises auf die Patenterteilung: **20.05.2026**
(21) Anmeldenummer: 17715507.4
(22) Anmeldetag: 10.04.2017
(51) Int. Cl.: A23K 20/121, A23L 19/00, A23B 2/733, A61K 36/21, A23L 33/105

(54) **ZUSAMMENSETZUNG UND HERSTELLUNG VON SPINATEXTRAKT MIT BETA-ECDYSON**
FORMULATION AND PRODUCTION OF SPINACH EXTRACT WITH BETA ECDYSON
COMPOSITION ET PRODUCTION D'EXTRAIT D'ÉPINARD AVEC DU BETA-ECDYSON

(30) Priorität: 11.04.2016 EP 16164749
(43) Veröffentlichungstag der Anmeldung: 20.02.2019
(73) Patentinhaber: Dr. Goetz GmbH, 34399 Wesertal (DE)
(72) Erfinder: ERFURT, Harry, 37170 Uslar (DE); STÜRTZ, Melanie, 37671 Höxter (DE); GÖTZ, Marcus Rudolf, 34399 Oberweser (DE); HILMER, Jens-Michael, 37603 Holzminden (DE); WINKLER, Dominik, 37671 Höxter (DE); DOSSI, Michael, 69502 Hemsbach (DE)
(74) Vertreter: Global IP Europe Patentanwaltskanzlei
(86) Internationale Anmeldenummer: PCT/EP2017/058586
(87) Internationale Veröffentlichungsnummer: WO 2017/178444

(56) Entgegenhaltungen:
- WO-A1-2015/151066
- CN-A- 104 844 676
- JP-B1- 5 723 476
- US-A- 4 923 697
- US-A1- 2008 138 393
- US-A1- 2012 196 818
- BERGMAN M ET AL: "The antioxidant activity of aqueous spinach extract: chemical identification of active fractions", PHYTOCHEMISTRY, PERGAMON PRESS, GB, vol. 58, no. 1, 1 September 2001 (2001-09-01), pages 143 - 152, XP004301205, ISSN: 0031-9422, DOI: 10.1016/S0031-9422(01)00137-6
- LAURA JAIME ET AL: "Extraction of functional ingredients from spinach ( Spinacia oleracea L.) using liquid solvent and supercritical CO 2 extraction : Extraction of functional ingredients from spinach", JOURNAL OF THE SCIENCE OF FOOD AND AGRICULTURE, vol. 95, no. 4, 21 July 2014 (2014-07-21), GB, pages 722 - 729, XP055671380, ISSN: 0022-5142, DOI: 10.1002/jsfa.6788
- RUDOLF EDENHARDER ET AL: "Isolation and Characterization of Structurally Novel Antimutagenic Flavonoids from Spinach ( Spinacia oleracea )", JOURNAL OF AGRICULTURAL AND FOOD CHEMISTRY, vol. 49, no. 6, 1 June 2001 (2001-06-01), US, pages 2767 - 2773, XP055304431, ISSN: 0021-8561, DOI: 10.1021/jf0013712
- ARITOMI M ET AL: "Three highly oxygenated flavone glucuronides in leaves of Spinacia oleracea", PHYTOCHEMISTRY, ELSEVIER, AMSTERDAM , NL, vol. 23, no. 9, 21 August 1984 (1984-08-21), pages 2043 - 2047, XP026606127, ISSN: 0031-9422, [retrieved on 19840821], DOI: 10.1016/S0031-9422(00)84967-5
- ' ARITOMI MASAKAZL ET AL: "FLAVONOL GLYCOSIDES IN LEAVES OF SPINACIA OLERACEA+", PHYTOCHEMISTRY, vol. 25, no. 1, 1 January 1986 (1986-01-01), pages 231 - 234, XP055304433
- ROBERT J GREBENOK ET AL: "Occurrence and levels of ecdysteroids in spinach", LIPIDS, SPRINGER-VERLAG, BERLIN/HEIDELBERG, vol. 26, no. 8, 1 August 1991 (1991-08-01), pages 666 - 668, XP035173876, ISSN: 1558-9307, DOI: 10.1007/BF02536433
- DATABASE WPI Week 201538, Derwent World Patents Index; AN 2015-31238E
- DATABASE WPI Week 201582, Derwent World Patents Index; AN 2015-61329X
- MI JIN CHO ET AL: "Flavonoid content and antioxidant capacity of spinach genotypes determined by high-performance liquid chromatography/mass spectrometry", JOURNAL OF THE SCIENCE OF FOOD AND AGRICULTURE, vol. 88, no. 6, 1 January 2008 (2008-01-01), GB, pages 1099 - 1106, XP055304165, ISSN: 0022-5142, DOI: 10.1002/jsfa.3206
- ELKE AEHLE ET AL: "Development and evaluation of an enriched natural antioxidant preparation obtained from aqueous spinach (Spinacia oleracea) extracts by an adsorption procedure", FOOD CHEMISTRY, vol. 86, no. 4, 1 August 2004 (2004-08-01), NL, pages 579 - 585, XP055304590, ISSN: 0308-8146, DOI: 10.1016/j.foodchem.2003.10.006
- SANTOS J ET AL: "Phenolic profile evolution of different ready-to-eat baby-leaf vegetables during storage", JOURNAL OF CHROMATOGRAPHY, ELSEVIER SCIENCE PUBLISHERS B.V, NL, vol. 1327, 4 January 2014 (2014-01-04), pages 118 - 131, XP028812504, ISSN: 0021-9673, DOI: 10.1016/J.CHROMA.2013.12.085

## Beschreibung

Die vorliegende Erfindung bezieht sich auf das Gebiet der Extrakte, genauer der Extrakte aus Spinat.

Spinat sowie Extrakte aus Spinat sind aufgrund ihrer vorteilhaften Eigenschaften von großem Interesse, nicht nur in der Nahrungsmittelindustrie, sondern auch bei Kosmetika, Nahrungsergänzungsmitteln, Tiernahrung oder Pharmazeutika. Der Stand der Technik beschreibt in JP 5 723476 B1 ein Verfahren zur Herstellung eines Spinatextrakt zur Anwendung in Nahrungsmitteln, hergestellt mittels der Extraktion von Spinatblättern mit wässrigen Ethanol und unter Anwendung eines Adsorptionsprozess an einem Styrol-Divinylbenzolpolymer Harz. Der β-Ecdyson-Gehalt des erhaltenen Konzentrats liegt unter 1% bezogen auf die Trockenmasse. US 2012/196818 A1 offenbart ein Verfahren zur Herstellung eines hochkonzentrierten Spinatextrakts zur Anwendung in Nahrungs- und Tierfuttermitteln, hergestellt mittels der Extraktion von Spinatblättern mit wässrigen Ethanol und unter Anwendung eines Adsorptionsprozess an einem Divinylbenzolpolymer Harz, wobei ein Extrakt mit hohem Polyphenolgehalt erhalten wird.

Es besteht somit eine ständige Notwendigkeit, die bestehenden Extrakte noch weiter zu verbessern.

Diese Aufgabe wird durch einen Extrakt gemäß Anspruch 1 gelöst. Demnach wird ein Spinatextrakt vorgestellt, enthaltend ≥ 1% bis ≤ 10% β-Ecdyson sowie ≥2% bis ≤50% Polyphenole, jeweils als Massenanteil im Trockenprodukt.

Unter dem Term "Spinatextrakt" wird insbesondere ein Stoffgemisch und/oder eine Zusammensetzung verstanden, welche aus Spinat (*Spinacia oleracea;* wird auch Echter Spinat, Gemüsespinat oder Gartenspinat genannt) oder Blanchierwasser zu dessen Haltbarmachung gewonnen wird. Dabei wird je nach Anwendung nach Extraktion und teilweisem oder völligem Eindampfen der Extraktionslösungsmittel (z.B. Wasser oder Ethanol) eine Extraktionslösung gewonnen, welche weiter durch verschiedene Verfahren getrocknet werden kann. Als Extrakt im Sinne der vorliegenden Erfindung werden aber auch Auszüge aus pflanzlichen Drogen verstanden, die u.a. durch Mazeration oder Perkolation mit Wasser oder Ethanol hergestellt werden.

Unter " β -Ecdyson" wird dabei 20-Hydroxy-Ecdyson mit der folgenden Struktur verstanden:

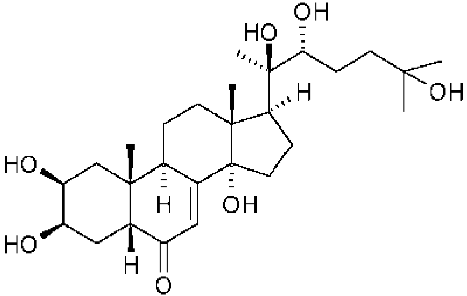

Diese Substanz ist unter anderem auch unter den Namen β-Ecdysone, 2β,3β,14α,20β,22,25-Hexahydroxy-7-cholesten-6-one, Ecdysterone, Insect moulting hormone, Polypodine A in der Literatur bekannt.

Unter Polyphenolen werden im Sinne der vorliegenden Erfindungen dabei insbesondere aromatische Verbindungen, die zwei oder mehr direkt an einen aromatischen Ring gebundene Hydroxygruppen enthalten, verstanden. Die meisten dieser Verbindungen gehören dabei zu den sekundären Pflanzenstoffen.

Überraschenderweise hat sich gezeigt, dass dieser Extrakt überwiegend ein sehr hohes antioxidatives Potential aufweist.

Bei den meisten Anwendungen der vorliegenden Erfindung kann einer oder können mehrere der folgenden Vorteile beobachtet oder erzielt werden:
- Besonders bevorzugt wird ein erfindungsgemäßer Extrakt aus sehr verdünnten Lösungen, Emulsionen oder Suspensionen (z.B. aus Nebenströmen der Lebensmittelproduktion) gewonnen.
- Der Extrakt liegt in einer stabilen Form vor und weist nur eine geringe Färbung auf, weshalb er auch gut zur Verwendung in weiteren Applikationen geeignet ist.
- Es handelt sich um einen standardisierten Extrakt, der einen definierten Gehalt an β-Ecdyson hat sowie eine vorteilhafte Flavonoid-Zusammensetzung,
- Die Löslichkeit des Extraktes ist sowohl in polaren Lösungsmitteln wie Wasser als auch in unpolaren Lösungsmitteln wie z.B. Pflanzenöl gegeben.
- Der Extrakt weist einen geringen Gehalt der Stoffe Nitrat/Nitrit sowie Oxalsäure auf.

Gemäß einer bevorzugten Ausführungsform der Erfindung beträgt der Gehalt an β-Ecdyson von ≥2% bis ≤8%, sowie am meisten bevorzugt ≥5% bis ≤7%. Dies hat sich bei den meisten Anwendungen besonders bewährt.

Gemäß einer bevorzugten Ausführungsform der Erfindung beträgt der Gehalt an Polyphenolen zwischen ≥10% bis ≤40%, sowie am meisten bevorzugt ≥25% bis ≤30%.

Gemäß einer bevorzugten Ausführungsform der Erfindung umfassen die Polyphenole Flavonoide.

Darunter werden insbesondere Verbindungen verstanden, die zwei aromatischen Ringe enthalten, die durch einen Tetrahydropyran-Ring verbunden sind.

Bevorzugt besteht dabei der Anteil der Flavonoide im Extrakt zwischen ≥10% bis ≤38%, noch bevorzugt ≥14% bis ≤32%, sowie am meisten bevorzugt ≥25% bis ≤30%.

Bevorzugt umfassen die Flavonoide dabei eine oder mehrere Verbindungen ausgewählt aus Glucuroniden, Di- oder Triglycosiden von methylierten und/oder methylendioxyderivatisierten 6-Oxo-Flavonolen.

Noch bevorzugt umfassen die Flavonoide dabei eine oder mehrere der folgenden Verbindungen:
5,3',4'-Trihydroxy-3-methoxy-6,7-methylenedioxyflavone 4'-glucuronid mit folgender Struktur: 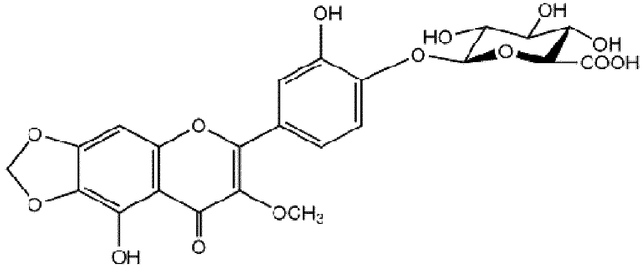
Jaceidin 4'-glucuronide = (3S,6S)-6-[4-(5,7-dihydroxy-3,6-dimethoxy-4-oxochromen-2-yl)-2-methoxyphenoxy]-3,4,5-trihydroxyoxane-2-Carbonsäure mit folgender Struktur: 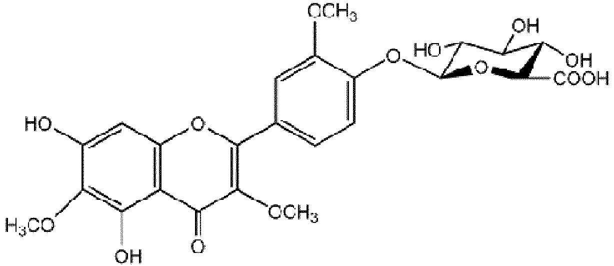
Spinacetin 3-gentiobiosid = 5,7-dihydroxy-2-(4-hydroxy-3-methoxyphenyl)-6-methoxy-3-[(2R,5S)-3,4,5-trihydroxy-6-[[(2R,4S,5S)-3,4,5-trihydroxy-6-(hydroxymethyl)oxan-2-yl]oxymethyl]oxan-2-yl]oxychromen-4-on mit folgender Struktur: 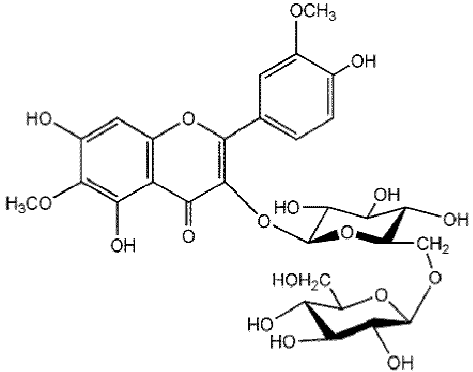
Isorhamnetin 3-sophoroside-7-glucosid = 3-[(2S,5S)-4,5-dihydroxy-6-(hydroxymethyl)-3-[(2S,3R,5S)-3,4,5-trihydroxy-6-(hydroxymethyl)oxan-2-yl]oxyoxan-2-yl]oxy-5-hydroxy-2-(4-hydroxy-3-methoxyphenyl)-7-[(2S,4S,5S)-3,4,5-trihydroxy-6-(hydroxymethyl)oxan-2-yl]oxychromen-4-on mit folgender Struktur: 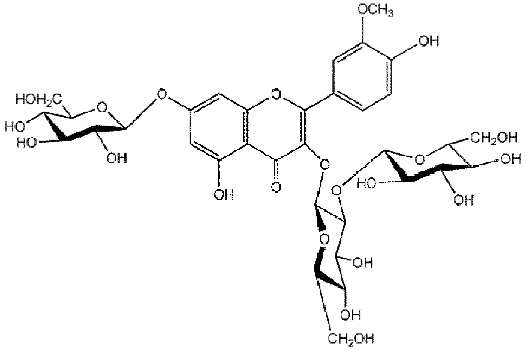
Spinatosid = 3,4',5,7-Tetrahydroxy-3',6-dimethoxyflavon mit folgender Struktur

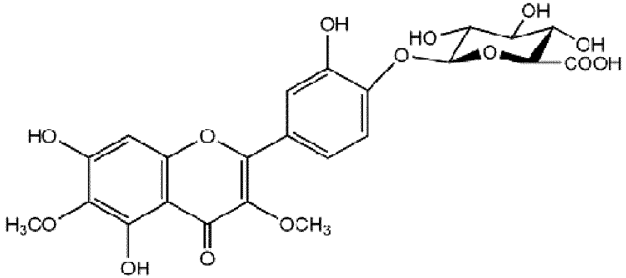

Bevorzugt umfasst dabei der Extrakt Spinatosid und 5,3',4'-Trihydroxy-3-methoxy-6:7-methylenedioxy-flavone-4'- O-glucuronid. Dies hat sich bei vielen Anwendungen bewährt, da so das enthaltende antioxidative Potential oftmals und überraschenderweise besonders hoch ist.

Dabei ist besonders bevorzugt, dass das Verhältnis von Spinatosid zu 5,3',4'-Trihydroxy-3-methoxy-6:7-methylenedioxy-flavone-4'- O-glucuronid zwischen ≥1:0,1 und ≤1:20 (Gew:Gew). Auch dies hat sich bei vielen Anwendungen bewährt, da so das enthaltende antioxidative Potential oftmals und überraschenderweise nochmals verstärkt wird.

Besonders bevorzugt beträgt das Verhältnis von Spinatosid zu 5,3',4'-Trihydroxy-3-methoxy-6:7-methylenedioxy-flavone-4'- O-glucuronid zwischen ≥1:0,5 und ≤1:8, noch bevorzugt ≥1:2 und ≤1:5.

Bevorzugt beträgt der addierte Anteil an Nitrat/Nitrit im Extrakt ≤500 ppm. Dies hat sich bei vielen Anwendungen der vorliegenden Erfindung bewährt.

Bevorzugt beträgt der addierte Anteil an Nitrat/Nitrit im Extrakt ≤400 ppm, noch bevorzugter ≤300 ppm.

Bevorzugt beträgt der Anteil an Oxalat im Extrakt ≤500 ppm. Auch dies hat sich als vorteilhaft herausgestellt.

Bevorzugt beträgt die Absorption von einer 0,5 Gew-%-Lösung des Extrakts in destilliertem Wasser bei 507nm ≤0,5. Dies hat sich bei vielen Anwendungen der vorliegenden Erfindung bewährt, da so der Extrakt aufgrund seiner relativen farblichen Neutralität bei vielen sensiblen Anwendungen eingesetzt werden kann.

Bevorzugt beträgt die Absorption von einer 0,5 Gew-%. Lösung des Extrakts in destilliertem Wasser bei 580nm ≤0,35. Auch dies hat sich bei vielen Anwendungen der vorliegenden Erfindung bewährt, da so der Extrakt aufgrund seiner relativen farblichen Neutralität bei vielen sensiblen Anwendungen eingesetzt werden kann.

Die vorliegende Erfindung bezieht sich ausserdem auf ein Arzneimittel enthaltend den erfindungsgemäßen Extrakt und/oder auf die Verwendung des erfindungsgemäßen Extrakts als Arzneimittel.

Überraschenderweise hat sich herausgestellt, dass der erfinderische Extrakt für die Behandlung der Muskelatrophie, Muskelhypotonie und Sarkopenie eingesetzt werden kann.

Die vorliegende Erfindung bezieht sich somit ausserdem auf ein Arzneimittel zur Behandlung der Muskelatrophie, Muskelhypotonie und/oder Sarkopenie, umfassend den erfindungsgemäßen Extrakt.

Weiterhin bezieht sich die vorliegende Erfindung auf eine Verwendung eines Arzneimittels, enthaltend den erfindungsgemäßen Extrakt zur Behandlung der Muskelatrophie, Muskelhypotonie und/oder Sarkopenie.

Weiterhin ist ein Verfahren zur Behandlung der Muskelatrophie, Muskelhypotonie und/oder Sarkopenie umfassen den Schritt - Verabreichen einer ausreichenden Menge eines Arzneimittels enthaltend den erfindungsgemäßen Extrakt an einen Patienten beschrieben.

Die Arzneimittel im Sinne der vorliegenden Erfindung sind vorzugsweise gebrauchsfertige pharmazeutische Zubereitungen, vorzugsweise in folgenden Formen: feste galenische Formen (wie z.B. Tabletten (mit Überzug und ohne, mit modifizierter Freisetzung und ohne), Dragees (mit Überzug und ohne, mit modifizierter Freisetzung und ohne), Kapseln (Weich- oder Hartgelatinekapseln mit modifizierter Freisetzung und ohne) Granulate (mit modifizierter Freisetzung und ohne), Pulver (mit modifizierter Freisetzung und ohne), Suppositorien (mit Überzug und ohne, mit modifizierter Freisetzung und ohne) Lutschbonbons, Kaugummis), sowie flüssige Formen (wie z.B. Lösungen, Suspensionen, Emulsionen, Sirupe (umgangssprachlich Hustensaft), Mundspülungen, Gurgellösungen, Halssprays oder Nasensprays, Nasentropfen, Nasenspüllösungen, Nasenpulver, Nasensalben oder Ohrentropfen, Ohrensprays, Ohrenspüllösungen, Ohrenpuder, Ohrentampons) sowie halbfeste Formen (wie z.B. Hydrophobe Salben darunter z.B.: Kohlenwasserstoffgele, Lipogele. Silikongele, Oleogele. sowie wasseraufnehmende Salben darunter z.B. Absorptionsbasen, hydrophile Salben, hydrophile Gele (Hydrogele) oder Pasten sowie Inhalate (wie z.B. Druckgas-Dosierinhalatoren, Pulver-Inhalatoren, Inhaltoren mit Zerstäuber, Inhalationskonzentrate zur Bereitung von Inhalationen) sowie wirkstoffhaltige Pflaster oder andere therapeutische Systeme.

Die erfindungsgemäßen pharmazeutischen Zubereitungen können (weitere) pharmazeutische Hilfs- und/oder Zusatzstoffe enthalten, wie sie üblicherweise in solchen Zubereitungen verwendet werden, z.B. Wirkstoffe sowie Füllstoffe (z.B. Cellulose, Calciumcarbonat), Fließ- und Rieselmittel (z.B. Talkum, Magnesiumstearat), Überzüge (z.B. Polyvinylacetatphtalat, Hydroxypropylmethylcellulosephtalat), Sprengmittel (z.B. Stärke, quervernetztes Polyvinylpyrrolidon), Weichmacher (z.B. Triethylcitrat, Dibutylphtalat) Stoffe zur Granulierung (Lactose, Gelatine), Retardierung (z.B. Poly(meth)acrylsäure-methyl/ethyl/2-trimethylaminoethylester-Copolymerisate in Dispersion, Vinylacetat/ Crotonsäure-Copolymerisate), Kompaktierung (z.B. Mikrokristalline Cellulose, lactose), Lösungs-, Suspendier oder Dispergiermittel (z.B. Wasser, Ethanol), Emulgatoren (z.B. Cetylalkohol, Lecithin), Stoffe zur Veränderung der rheologischen Eigenschaften (Siliciumdioxid, Natriumalginat), Stoffe zur mikrobiellen Stabilisierung (z.B. benzalkoniumchlorid, Kaliumsorbat), Konservierungsmittel und Antioxidantien (z.B. DLalpha-tocopherol, Ascorbinsäure), Stoffe zur Veränderung des pHs (Milchsäure, Citronensäure), Treib- oder Inert-Gase (z.B. Fluorierte Chlorkohlenwasserstoffe, Kohlendioxid), Farbstoffe (Eisenoxide, Titandioxid), Salbengrundstoffe (z.B. Paraffine, Bienenwachs), u.a. wie sie in der Fachliteratur (z.B. Schmidt, Christin. Wirk- und Hilfsstoffe für Rezeptur, Defektur und Großherstellung. 1999; Wissenschaftliche Verlagsgesellschaft mbH Stuttgart oder Bauer, Frömming Führer. Lehrbuch der Pharmazeutischen Technologie. 8. Auflage, 2006. Wissenschaftliche Verlagsgesellschaft mbH Stuttgart).

Die jeweils einzusetzenden Mengen können in Abhängigkeit von der Art des jeweiligen Produkts vom Fachmann durch einfaches Ausprobieren leicht ermittelt werden.

Die vorliegende Erfindung bezieht sich außerdem auf eine Aromamischung umfassend den erfindungsgemäßen Extrakt in einer Menge von 0,1 bis 5 Gew.-%.

Die vorliegende Erfindung bezieht sich weiterhin auf konfektionierte Produkte umfassend den erfindungsgemäßen Extrakt.

Diese können dabei Nahrungsmittel und/oder Nahrungsergänzungsmittel und/oder entsprechende Produkte für die Tierernährung und/oder Tiergesundheit sein.

Soweit die konfektionierten Produkte Nahrungsmittel darstellen, denen man den Extrakt direkt zusetzt, handelt es sich beispielsweise um Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (karbonisierte) fruchthaltige Limonaden, (karbonisierte) isotonische Getränke, (karbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchproteinhaltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die antioxidativen Zubereitungen oder die Extrakte können insbesondere in Sportgetränken eingesetzt werden, darunter insbesondere in solchen Sportgetränken, die der Regeneration des Sportlers nach einer intensiven sportlichen Tätigkeit dienen oder die die Leistungsfähigkeit steigern.

Der Extrakt wird dabei üblicherweise in Mengen von etwa 0,1 bis 5, vorzugsweise etwa 0,5 bis 3 und insbesondere etwa 1 bis 2 Gew.-% zugesetzt.

### 1. Kapseln

Handelt es sich bei den Produkten um Nahrungsmittelergänzungsstoffe, so werden diese in der Regel ohne weitere Zusatzstoffe eingesetzt, wobei von reinen Konfektionierungsmitteln abzusehen ist. Eine bevorzugte Anwendungsform stellen hier Makro- oder Mikrokapseln dar. Makrokapseln bestehen vorzugsweise aus Gelatine. Diese weisen in der Regel Teilchendurchmesser von 0,5 bis 1,5 cm auf. Oder es handelt sich um sprühgetrocknete Produkte, die als Basis Polysaccharide oder Dextrine enthalten.

Unter den Begriffen "Mikrokapsel" oder "Nanokapsel" werden vom Fachmann hingegen sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon. Beispiele für Mikrokapseln des Stands der Technik sind folgende Handelsprodukte (in Klammern angegeben ist jeweils das Hüllmaterial): Hallcrest Microcapsules (Gelatine, Gummi Arabicum), Coletica Thalaspheres (maritimes Collagen), Lipotec Millicapseln (Alginsäure, Agar-Agar), Induchem Unispheres (Lactose, mikrokristalline Cellulose, Hydroxypropylmethylcellulose); Unicerin C30(Lactose, mikrokristalline Cellulose, Hydroxypropylmethylcellulose), Kobo Glycospheres (modifizierte Stärke, Fettsäureester, Phospholipide), Softspheres (modifiziertes Agar-Agar) und Kuhs Probiol Nanospheres (Phospholipide) sowie Primaspheres und Primasponges (Chitosan, Alginate) und Primasys (Phospholipide).

### 2. Kaugummis

Eine weitere Darreichungsform der Extrakte kann in Form von Kaugummis erfolgen. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren.

Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500 , auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener/Weichmacher, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 (Wrigleys) sowie Stevia Extrakte und deren aktiven Bestandteile, insbesondere Rebaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 Gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

Bei den bevorzugten oralen Zubereitungen kann es sich auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol: Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500.

Zusätzlich zu der wasserunlöslichen Gummibasis enthalten Kaugummizubereitungen regelmäßig einen wasserlösliche Anteil, der beispielsweise von Weichmacher, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Weichmacher oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine. Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 Gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### Kosmetische Mittel

Ein weiterer Gegenstand der Erfindung betrifft kosmetische Mittel, enthaltend einen erfindungsgemäßen Extrakt

Die erfindungsgemäßen kosmetischen Mittel können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. Da viele pharmazeutische Zubereitungen ähnliche Inhaltsstoffe aufweisen, gelten die nachfolgenden Beispiele hier mit.

### 1. Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono - und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### 2. Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C6-C22-Fettsäuren mit linearen oder verzweigten C6-C22-Fettalkoholen bzw. Ester von verzweigten C6-C13-Carbonsäuren mit linearen oder verzweigten C6-C22-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C6-C22-Fettsäuren mit verzweigten Alkoholen, insbesondere 2-Ethylhexanol, Ester von C18-C38-Alkylhydroxycarbonsäuren mit linearen oder verzweigten C6-C22-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C6-C10-Fettsäuren, flüssige Mono-/Di-/Triglyceridmischungen auf Basis von C6-C18-Fettsäuren, Ester von C6-C22-Fettalkoholen und/oder Guerbetalkoholen mit aromatischen Carbonsäuren, insbesondere Benzoesäure, Ester von C2-C12-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C6-C22-Fettalkoholcarbonate, wie z.B. Dicaprylyl Carbonate (Cetiol<^{®}>CC), Guerbetcarbonate auf Basis von Fettalkoholen mit 6 bis 18, vorzugsweise 8 bis 10 C Atomen, Ester der Benzoesäure mit linearen und/oder verzweigten C6-C22-Alkoholen (z.B. Finsolv^{®} TN), lineare oder verzweigte, symmetrische oder unsymmetrische Dialkylether mit 6 bis 22 Kohlenstoffatomen pro Alkylgruppe, wie z.B. Dicaprylyl Ether (Cetiol<^{®}>OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### 3. Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia<^{®}>SP von Cognis;
- Polyalkylenglycole sowie
- Glycerincarbonat.

Im Folgenden werden besonders geeignete Emulgatoren näher erläutert:
(i) Alkoxylate.Die Anlagerungsprodukte von Ethylenoxid und/oder von Propylenoxid an Fettalkohole, Fettsäuren, Alkylphenole oder an Rizinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C12/18-Fettsäuremono- und -diester von Anlagerungsprodukten von Ethylenoxid an Glycerin sind als Rückfettungsmittel für kosmetische Zubereitungen bekannt.
(ii) Alkyl- und/oder Alkenyloligoglykosid.Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.
(iii) Partialglyceride.Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.
(iv) Sorbitanester.Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.
(v) Polyglycerinester. Typische Beispiele für geeignete Polyglycerinester sind Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Admul^{®} WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.
(vi) Anionische Emulgatoren. Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.
(vii)Amphotere und kationische Emulgatoren.Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C8/18-Alkyl- oder Acylgruppe im Molekül mindestens eine freie Aminogruppe und mindestens eine -COOH- oder -SO3H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C12/18-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### 4. Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### 5. Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### 6. Kühlstoffe

Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS<1>3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:
Beispiele für Anwendungen dieser Stoffe finden sich beispielsweise in den Druckschriften WO 2003 043431 (Unilever) oder EP 1332772 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat<^{®}>MGC), Menthol Propylenglycol Carbonate (FEMA GRAS 3784 = Frescolat<^{®}>MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat<^{®}>ML) und insbesondere das Menthone Glyceryl Acetal (FEMA GRAS 3807) bzw. Menthone Glyceryl Ketal (FEMA GRAS 3808), das unter der Bezeichnung Frescolat<^{®}>MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat<^{®}>MGA, Frescolat<^{®}>ML, Frecolat<^{®}>MGC und Frescolat<^{®}>MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

### 7. Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und - diester von Fettsäuren, Polyacrylate, (z.B. Carbopole^{®} und Pemulen-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone<^{®}>Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### 8. Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium - und/oder Zinkstearat bzw. -ricinoleat eingesetzt werden.

### 9. Polymere

Geeignete kationische Polymere sind beispielsweise kationische Cellulosederivate, wie z.B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium Hydroxypropyl Hydrolyzed Collagen (Lamequat^{®}L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine^{®}/Sandoz), Copolymere der Acrylsäure mit Dimethyl-diallylammoniumchlorid (Merquat^{®} 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ AcrylatCopolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/VinylacetatCopolymere, Vinylpyrrolidon/Dimethylaminoethylmethacrylat/Vinylcaprolactam-Terpolymere sowie gegebenenfalls derivatisierte Celluloseether und Silicone in Frage.

### 10. Silikonverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### 11. UV-Lichtschutzfaktoren

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl- hexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexyl-ester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb^{®} HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan<^{®}>AP)
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol^{®} 1789), 2-(4-Diethylamino-2-hydroxybenzoyl)-benzoic acid hexylester (Uvinul^{®} A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol^{®} 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex<^{®}>T2000, Eusolex<^{®}>T, Eusolex<^{®}>T-ECO, Eusolex<^{®}>T-S, Eusolex<^{®}>T-Aqua, Eusolex<^{®}>T-45D (alle Merck), Uvinul TiO2(BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE<^{®}>oder Z-COTE HP1<^{®}>verwendet.

### 12. Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### 13. Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen insbesondere die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl - und Lauryl-, Palmitoyl-, Oleyl-, ã-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. VitaminE-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure,

Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### 14. Hydrotrope

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschilderten Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glutamin;
- Dialkoholamine, wie Diethanolamin oder 2-Amino-1,3-propandiol.

### 15. Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine<^{®}>bekannten Silberkomplexe und die in Anlage 6, Teil A und B der Kosmetikverordnung aufgeführten weiteren Stoffklassen.

### 16. Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen A-nethol, Citronellol, Eugenol, Isoeugenol, Geraniol, Linalool, Phenylethylalkohol und Terpineol, zu den Kohlenwasserstoffen gehören hauptsächlich die Terpene und Balsame.

Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### 17. Farbstoffe

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

Die konfektionierten Produkte können, wie beschrieben, auch Tiernahrungsmittel oder Mittel für die Tiergesundheit sein. Dabei können alle oben beschriebenen weiteren Ausführungsformen oder Ausgestaltungen, je nach Ausführungsform der vorliegenden Erfindung mit verwendet werden.

Unter Tiernahrung ist hierbei jegliche flüssige oder feste, zur Aufnahme durch das Tier bestimmte Nahrung zu verstehen. So kommen beispielsweise in Betracht Lösungen oder Suspensionen in Wasser, Milch oder Mischungen mit festen Nahrungsstoffen, wie z.B. Kleie, Mehl, Fleisch, Faserstoffe usw.

Gemäß einer bevorzugten Ausführungsform der vorliegenden Erfindung enthalten die Tiernahrungsmittel noch sogenannte Hydrolysate. Der Begriff Hydrolysat bezeichnet das Produkt, das aus der Hydrolyse eines Substrats führt oder entsteht. Die Auswahl eines geeigneten Substrats wird von den gewünschten Eigenschaften des Hydrolysats am Ende des Prozesses, und zwar im Hinblick auf die organoleptischen Eigenschaften und Nährwerte erreicht bestimmt. Das Substrat ist vorzugsweise ein nicht-Milchproteinsubstrat, bevorzugter ein tierisches Eiweiß, noch stärker bevorzugt Gewebe von Nutztieren wie Geflügel (z.B. jede Spezies oder Art von Vogel, vorzugsweise Huhn, Pute oder Ente), Rind, Schwein oder Lamm oder von einem Meeresfrüchte Tier wie Garnelen, Fisch oder Muscheln. In einer besonders bevorzugten Ausführungsform ist das Substrat Eingeweide aus Huhn. Ein Hydrolysat von tierischem Protein kann auch ein Verdauungsprodukt aus tierischem Gewebe sein.

In einer Ausführungsform der vorliegenden Erfindung werden die tierische Proteine, aus welchem die Hydrolysate hergestellt werden, aus Eingeweiden aus geeigneten Quellen erhalten. Typischerweise umfassen Eingeweide die weichen inneren Organe des Körpers, zum Beispiel die Lunge, Milz, Nieren, Gehirn, Leber, Tieftemperatur entfettete Gewebe, sowie Mägen und Därme, befreit von ihrer Inhalte; insbesondere jene Organe innerhalb der Bauch- und Brusthöhle enthielt. Zusätzlich oder alternativ zu weichen inneren Organe können Eingeweide sind Blut und / oder Knochen. Ein Beispiel für die Definition von Eingeweiden wird durch die Association of American Feed Control Officals, Inc. (AAFCO) gegeben. Die AAFCO definiert Eingeweide wie alle Organe in den drei großen Hohlräume des Körpers (Bauch-, Brust- und Becken), jedoch Eingeweide für Fische als solche Organe in der großen Hohlraum des Körpers, einschließlich der Kiemen, Herz, Leber, Milz, Magen und Darm. Ebenso definiert AAFCO Eingeweide für Säugetiere als die Organe in der großen Hohlraum des Körpers, einschließlich der Speiseröhre, Herz, Leber, Milz, Magen und Darm, schließt aber den Inhalt des Verdauungstraktes aus und definiert Eingeweide für Geflügel als solche Organe in dem großen Hohlraum des Körpers, einschließlich der Speiseröhre, Herz, Leber, Milz und Magen sowie unentwickelte Eier und Darm. In verschiedenen Ausführungsformen können die Eingeweide vorbehandelt sein, beispielsweise durch Rühren, Homogenisieren, Emulgieren und dergleichen. Solche Beispiele sind dem Fachmann leicht zugänglich.

Die vorliegende Erfindung bezieht sich außerdem auf ein Verfahren zur Herstellung eines Spinatextrakts, umfassend die Schritte:
a) Vorbehandeln des Spinats durch Blanchieren des Spinats mit Wasser oder einer wässrigen Lösung bei einer Temperatur von ≥ 70°C für eine Zeitdauer von ≤ 10 Minuten, unter Erhalt eines Blanchierwassers;
b) Durchführung einer Adsorption an einem Adsorberharz, worin das Adsorberharz ausgewählt ist aus der Gruppe, die besteht aus Copolymerisaten aus Ethylvinylbenzol und Divinylbenzol, Vinylpyrrolidon und Divinylbenzol, Vinylpyridin und Divinylbenzol, Styrol und Divinylbenzol, Polyaromaten, Polystyrol, Poly(meth)aycrylaten, Polypropylen, Polyestern, Polytetraflourethylen und Mischungen dieser Verbindungen.;
c) Elution mittels eines Lösemittels mit einem E_{T}30-Wert von ≥30 oder mit einem Lösemittelgemisch, enthaltend mindestens ein Lösemittel mit einem E_{T}30-Wert von ≥ 30; und
d) Aufkonzentrierung Überraschenderweise hat sich herausgestellt, dass auf diese Weise bei den meisten Anwendungen der Erfindung ein Extrakt mit hervorragenden Eigenschaften erlangt werden kann. Insbesondere kann der oben beschriebene Extrakt auf diese Weise gewonnen werden.

Weiterhin weist bei den meisten Anwendungen der Erfindung der Extrakt eine oder mehrere der folgenden Vorteile und Eigenschaften auf:
- Besonders bevorzugt wird ein erfindungsgemäßer Extrakt aus sehr verdünnten Lösungen, Emulsionen oder Suspensionen (z.B. aus Nebenströmen der Lebensmittelproduktion) gewonnen.
- Der Extrakt weist einen günstigen Gehalt von β-Ecdyson und Polyphenolen auf.
- Der Gehalt an Nitrat/Nitrit und/oder Oxalat ist dagegen gering
- Der Extrakt weist eine gute Löslichkeit auf und kann damit für verschiedenste Applikationen verwendet werden
- Die Farbe des resultierenden Extraktes ist nicht besonders stark ausgeprägt

Die einzelnen Schritte des Verfahrens werden im Folgenden erläutert, wobei die einzelnen vorteilhaften Ausgestaltungen sowie Einzelheiten des Verfahrens beliebig miteinander kombinierbar sind und ggf. isoliert einzelne bevorzugte Ausführungsformen der vorliegenden Erfindung darstellen.

### Schritt a)

Der Term "Vorbehandeln des Spinats" beinhaltet und/oder bedeutet insbesondere, dass Spinat so aufbereitet wird, dass anschließend die Schritte b) und c) durchführbar sind.

Dabei beinhaltet Schritt a) Blanchieren mit Wasser oder wässriger Lösung.

Dabei bedeutet der Term "Blanchieren" insbesondere das Behandeln des Spinats mit Wasser oder wässriger Lösung bei einer Temperatur von ≥ 70°C, bevorzugt ≥ 90°C für ≥10 Sekunden, bevorzugt aber ≤ 10 Minuten. Bevorzugte Blanchierzeiten sind ≥20 Sekunden, bevorzugt ≥1 bis ≤ 2 Minuten.

Das Blanchieren kann mit destilliertem Wasser, aber auch Leitungs- oder Quellwasser erfolgen.

Bevorzugt beträgt dabei das Verhältnis von Spinat zu Wasser (in g/l) von ≥ 50 g/l bis ≤ 800 g/l.

Schritt a) findet unter Verwendung von Wasser oder einer wässrigen Lösung statt.

Bevorzugte weitere Inhaltsstoffe sind dabei Salze, insbesondere Kochsalz sowie Antioxidantien wie Ascorbinsäure oder Mischungen daraus.

Alternativ oder zusätzlich kann Schritt a) beinhalten, dass der Spinat mit Wasser oder wäßriger Lösung versetzt und dann homogenisiert wird. Bevorzugt wird danach filtriert.

### Schritt b)

Unter dem Term "Adsorption" wird insbesondere verstanden, dass das zu extrahierende Material einem geeigneten Adsorberharz ausgesetzt wird.

Schritt b) findet unter Verwendung des Blanchierwassers statt, wobei die bevorzugten Inhaltsstoffe wie oben angegeben sind.

Dies geschieht bevorzugt bei ≥ 10 °C bis ≤ 60 °C, dabei noch bevorzugt bei ≥ 20 °C bis ≤ 30 °C, am meisten bevorzugt bei Raumtemperatur.

Die Adsorptionsmaterialien sind ausgewählt aus der Gruppe umfassend verschiedenartig vernetzte Polystyrole, bevorzugt Copolymerate aus Ethylvinylbenzol und Divinylbenzol, Vinylpyrrolidon und Divinylbenzol, Vinylpyridin und Divinylbenzol, Styrol und Divinylbenzol, Polyaromate, Polystyrole, Poly(meth)acrylate, Polypropylene, Polyester, Polytetrafluorethylen oder Mischungen dieser Verbindungen.

Gemäß einer bevorzugten Ausführungsform der Erfindung ist dabei die durchschnittliche Porengröße des Adsorbermaterials von ≥ 2 nm bis ≤ 50 nm, bevorzugt ≥5 nm bis ≤40 nm.

Gemäß einer bevorzugten Ausführungsform der Erfindung beträgt die Oberfläche (in m²/g) des Adsorbermaterials von ≥ 300 m²/g bis ≤ 1200 m²/g, bevorzugt ≥ 400 m²/g bis ≤ 900 m²/g. Besonders bevorzugt sind dabei die unter den Handelsnamen LEWATIT^{®} (z.B. LEWATIT^{®} 1064 OC),; TREVER ^{®} I SORB (z.B. TREVER^{®} I SORB ADS 400, TREVER^{®} I SORB ADS 700, TREVER^{®} I SORB ADS 800), RENSA^{®} (z.B. RENSA^{®} PY), STRATA-X^{™}, AMBERCHROM^{™} (z.B: AMBERCHROM^{™} CG 300m, AMBERCHROM^{™} CG 300c), AMBERLITE^{™} (z.B: AMBERLITE^{™} XAD^{™} 7HP) erhältlichen Substanzen.

In einer bevorzugten Ausgestaltung der Erfindung wird die Adsorption unter Durchfluss durchgeführt, in dem das zu extrahierende Material mit dem Absorptionsmaterial in Kontakt gebracht wird. Dabei wird bevorzugt das Bettvolumen pro Stunde (BV/h) auf einen Wert zwischen ≥1 BV/h bis ≤30 BV/h, bevorzugt ≥15 BV/h bis ≤20 BV/h festgelegt.

### Schritt c)

Unter "Elution" wird insbesondere verstanden, dass das Adsorptionsmaterial aus Schritt b) nach Durchführung der Adsorption einem anderen Lösemittel als in Schritt b) ausgesetzt wird, so dass die an das Adsorptionsmaterial gebundenen Stoffe zumindest teilweise wieder freigesetzt (= eluiert) bzw. desorbiert werden.

Unter "E_{T}(30)-Wert" wird die Polarität eines Lösemittels verstanden, wobei sich im Rahmen der vorliegenden Erfindung auf die Werte bezogen wird, welche in Reichart; Dimroth Fortschr. Chem. Forsch. 1969, 11, 1-73, Reichart Angew. Chem. 1979, 91, 119 -131 sowie zitiert in March, Advanced Organic Chemistry, 4. Auflage, J. Wiley & Sons, 1992, Tabelle 10.13, S.361 veröffentlicht worden sind.

Wenn ein Lösemittelgemisch vorliegt, weisen bevorzugt dann alle Lösemittel einen E_{T}30-Wert von ≥30 auf.

Gemäß einer bevorzugten Ausführungsform der Erfindung wird die Elution mittels eines Lösemittels mit einem E_{T}30-Wert von ≥45 oder mit einem Lösemittelgemisch, enthaltend mindestens ein Lösemittel mit einem E_{T}30-Wert von ≥45 durchgeführt.

Wenn ein Lösemittelgemisch vorliegt, weisen bevorzugt dann alle Lösemittel einen E_{T}30-Wert von ≥45 auf.

Bevorzugte Lösemittel in Schritt c) sind dabei Alkohole, insbesondere Ethanol, Methanol 1-Propanol, 2-Propanol, weiterhin Ethylacetat, Acetonitril, DMSO und Mischungen dieser Lösemittel entweder untereinander, mit anderen Lösemitteln oder mit Wasser und/oder wässriger Lösung.

In einer bevorzugten Ausgestaltung der Erfindung wird die Adsorption unter Durchfluss durchgeführt, in dem das zu extrahierende Material an dem Absorptionsmaterial vorbeigeführt wird. Dabei wird bevorzugt das Bettvolumen pro Stunde (BV/h) auf einen Wert zwischen ≥ 0,1 BV/h bis ≤ 10 BV/h, bevorzugt ≥ 2 BV/h bis ≤ 4 BV/h festgelegt.

### Schritt d) Aufkonzentrierung

Die Aufkonzentrierung kann bevorzugt durch Entfernen des Lösemittels erfolgen, beispielsweise durch Abdestillieren oder Verdampfen des Lösemittels.

Gemäß einer bevorzugten Ausführungsform der vorliegenden Erfindung können sich noch weitere Schritte anschließen, darunter insbesondere und insoweit bevorzugt Gefriertrockung.

Die vorliegende Erfindung bezieht sich außerdem auf einen Extrakt, erhältlich durch das Verfahren wie oben beschrieben.

Weiterhin kann ein gemäß dem Verfahren gewonnener Extrakt auf alle zuvor beschriebenen Weisen und in allen zuvor beschriebenen Produkten verwendet werden.

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus den Unteransprüchen sowie aus der nachfolgenden Beschreibung der Beispiele und Figuren, die rein illustrativ und nicht beschränkend zu verstehen sind.

Dabei zeigt:
- Fig. 1: ein Diagramm zeigend die Aktivierung von PI3K in Mäusezellen durch Behandlung mit mehreren Versuchslösungen,
- Fig. 2: ein Diagramm zeigend die Aktivierung von AKT in Mäusezellen durch Behandlung mit mehreren Versuchslösungen; sowie
- FIg. 3: der Zuwachs an Protein in Mäusezellen bei Behandlung mit mehreren Versuchslösungen.

### Beispiel I: Extraktion von homogenisiertem Spinat

Das Pflanzenmaterial (frischer Blattspinat vom lokalen Wochenmarkt) wurde vor der Extraktion entsprechend vorbereitet, in dem die einzelnen Spinat-Blätter in ca. 1 x 1 cm große Stücke geschnitten wurden. Dazu wurde das komplette Blatt samt Rippe verwendet.

50,89 g vorbereitete Spinatblätter wurden eingewogen und mit 200 mL Bi-dest. Wasser versetzt. Danach erfolgte eine Homogenisierung des Pflanzenmaterials mittels eines Ultraturrax^{®} (Stufe 4) für 2 Minuten und Kühlung auf ca. 25°C. Das auf diese Weise entstandene Homogenisat wurde für 15 Minuten unter Ultraschall behandelt (Ultraschallbad), wobei eine Temperierung auf 25°C vorgenommen wird. Im Anschluss wurde das Gemisch für 10 Minuten bei 4500 rpm zentrifugiert, bevor der Überstand über einen Faltenfilter in Eiswasser filtriert wurde.

Vom so erhaltenen Rohextrakt wurden 150 mL auf eine mit Wasser gespülte Amberlite^{™} XAD^{™} 7 Säule verbracht und in einer Geschwindigkeit von ca. 1 Tropfen/5 sec über die Säule gegeben. Das XAD-7 Material (Amberlite^{™} XAD^{™} 7HP) ist ein "moderately polar acrylic resin" mit einer Porengröße von 20-60 mesh. Gespült wurde die Säule mit 200 mL bidest. Wasser, bevor mit Ethanol eluiert wurde. Bei der Elution wurde mit leicht erhöhter Geschwindigkeit gearbeitet (ca. 3 Tropfen/10 sec). Ein klares, grün-braunes Eluat wurde erhalten.

Die Aufkonzentrierung dieses Eluates erfolgt bei 40°C unter reduziertem Druck. Danach schließt sich eine schonende Gefriertrocknung an, um einen "angereicherten Spinatextrakt" zu erhalten.

Anschließend wurde der Extrakt analysiert und folgende Anteile erhalten:
β-Ecdysone: ca. 0,0025-0,0030%
Gesamt- Polyphenol-Anteil: ca. 0,02%
5,3',4'-Trihydroxy-3-methoxy-6:7-methylenedioxy-flavone-4'-O-glucuronid/
Spinatosid: als Summe ca. 0,016% in einem Verhältnis ca. 4:1.

Der ORAC-Wert dieses Extraktes beträgt 860 µmol TE/g.

### Beispiel II: Extraktion von blanchiertem Spinat

Das Pflanzenmaterial (frischer Blattspinat z.B. Pistella/Italien) wird vor Extraktion entsprechend vorbereitet, in dem die einzelnen Spinat-Blätter in ca. 1 x 1 cm große Stücke geschnitten werden. Dabei wird das komplette Blatt samt Rippe verwendet.

Die Einwaage beträgt 30,42 g Blattware, welche in 500 mL kochendes (ca. 95°C) bidestilliertes Wasser für exakt 2 min verbracht wurde. Im Anschluss wurde das Blattmaterial schnell über einen Faltenfilter filtriert und das Blanchierwasser in einem Eisbad abgeschreckt. Vom so erhaltenen Blanchierwasser wurden 250 mL auf eine mit Wasser gespülte Amberlite^{™} XAD^{™} 7 Säule verbracht und in einer Geschwindigkeit von ca. 1 Tropfen/5 sec über die Säule gegeben.

Das XAD-7 Material (Amberlite^{™} XAD^{™} 7HP) ist ein "moderately polar acrylic resin" mit einer Porengröße von 20-60 mesh. Gespült wurde die Säule mit 200 mL bidest. Wasser, bevor mit Ethanol eluiert wurde. Die Aufkonzentrierung des Eluates erfolgte bei 40°C unter reduziertem Druck.

Anschließend wurde der Extrakt analysiert und folgende Anteile erhalten:
β-Ecdysone: 0,004%
Gesamt- Polyphenol-Anteil: 0,0012%
5,3',4'-Trihydroxy-3-methoxy-6:7-methylenedioxy-flavone-4'-O-glucuronid, Spinaosid: als Summe ca. 0,01% in einem Verhältnis von ca. 5:1
Der ORAC-Wert dieses Extraktes beträgt 650 µmol TE/g.

### Beispiel III: Extraktion in größerem Maßstab.

500 Liter Spinat-Blanchierwasser werden auf eine vorher mit Wasser gespülte Säule verbracht, die mit Adsorberharz (Lewatit) gefüllt ist. Dies erfolgt mit einem Volumenstrom von 30 Liter/h.

Anschließend wurde die Säule mit 30 Liter/h Wasser gespült, bevor mit 6 Liter/h Ethanol eluiert wurde.

Das erhaltene Produkt wurde daraufhin am Rotationsverdampfer von einem großen Anteil des Lösemittels befreit. Das Konzentrat wurde anschließend weiter getrocknet mittels Gefriertrocknung bis auf einen Wassergehalt von < 5 %. Es wurden auf diese Weise 58 g Produkt erhalten. Die Restfeuchte betrug unter 4 %.

Anschließend wurde der Extrakt analysiert und folgende Anteile erhalten:
β-Ecdysone: 3%
Gesamt- Polyphenol-Anteil: 15%
5,3',4'-Trihydroxy-3-methoxy-6:7-methylenedioxy-flavone-4'- O-glucuronid: 4,3% Spinaosid:1,1%

Der ORAC-Wert dieses Extraktes beträgt 3200 µmol TE/g.

### Vergleichsbeispiel:

Zum Vergleich wird ein ORAC- Wert des unter dem Namen "Auropure" erhältlichen Spinatextrakts gemessen. Dieser liegt bei ca. 300 µmol TE/g

### Beispiel IV: Wirkung des erfindungsgemäßen Extrakts

Zur Untersuchung der therapeutischen Wirkung des erfindungsgemäßen Extrakts wurde die Aktivierung des PKB/Akt Signalübertragungswegs untersucht. Dieser Signalübertragungsweg ist zentral für Muskelaktivierung und Muskelregeneration.

### Zellkulturen:

C2C12 Mausmuskelzellen wurden von Sigma-Aldrich (Taufkirchen, Deutschland) erhalten und in DMEM + 2mM Glutamine (InVitrogen/Thermo-Fisher Scientific) + 10% Fetal Bovine Serum FBS / FCS (Bio&Sell, Feucht, Germany) aufbewahrt.

### Proteinbestimmung

Die zu untersuchenden Zellen wurden für 8 h mit 100 µg/ml IGF-1 (Immunotools, Friesoythe, Germany (bekannter Aktivator für den PKB/Akt Signalübertragungswegs, positive Kontrolle) sowie einer Leerprobe (negative Kontrolle) behandelt.

Für die Bestimmung des Effekts des erfindungsgemäßen Extrakts wurden Zellen mit 1, 10 und 100 µg/ml Extraktlösung unter Verwendung des Extrakts lt. Beispiel III für 8 h behandelt.

Nach dem Behandeln wurden die Zellen mit kalter phosphatgepufferter Salzlösung behandelt und dann mit Lysepuffer (42 mM Tris-HCl, 1,3% SDS, 6,5% Glycerin, 100 µM Natriumorthovanadat und 2% Phosphatase und Protease inhibitoren) lysiert.

Der Proteingehalt wurde mittels des Bicinchoninsäure-Proteinbestimmungskits (BCA Protein Assay Kit, Thermo Fisher Scientific, Bonn, Germany) gemäß den Herstellerinstruktionen bestimmt. Für das Western Blotting wurden 10-20 µg des Proteins jeder Probe einer SDS-PAGE unter reduzierenden Bedingungen unterworden. Anschließend wurden die Proteine auf eine Polyvinylidenefluoridmembran (PVDF-Membran, Merck Millipore, Darmstadt, Germany) transferiert.

Nach Blockierung mit 5% Milchlösung (BioRad, Munich, Germany) in Tris-Pufferlösung (TBS) enthaltend 0.1% Tween 20 (TBS-T) wurden die Membranen mit den jeweiligen primären Antikörper über Nacht bei 4°C inkubiert. Die Antikörper wurden dabei in TBS-T mit 5% BSA verdünnt.

Die verwendeten primären Antikörper sind dabei in der folgenden Tabelle aufgeführt:

| **Zielprotein** | **Primärer Antikörper, Quelle** | **Verdünnung** | **Quelle** |
|---|---|---|---|
| | Phospho-PI3K p85 (Y458), | 1:1000 | Kaninchen |
| PI3K | Cells Signaling 9212S | | |
| | Phospho-Akt (S473), | 1:1000 | Kaninchen |
| pAKT | Cells Signaling 9271S, Probe 14 | | |
| | β-Aktin (C4), | 1:5000 | Maus |
| Aktin | Santa Cruz sc 47778, Probe L 1615 | | |

Nach intensivem Waschen (3 mal für 15 Minuten in TBS Lösung, enthaltend 0,1% Tween 20) wurden die Membranen für 1h mit dem sekundären Antikörper inkubiert, entweder Meerrettichperoxidase-gekoppelter anti-Maus IgG (HRP-coupled anti-mouse IgG) oder Meerrettichperoxidase-gekoppelter anti-Kaninchen IgG (HRP-coupled anti-rabbit IgG) (beide Amersham / GE Healthcare, Freiburg, Germany). Densitometrische Analyse wurde mittels der ImageJ software (NIH, USA) durchgeführt, wobei β-Aktin als Kontrolle benutzt wurde, um gleichmäßige Beladung der Proben und die Normalisierung der Daten sicherzustellen.

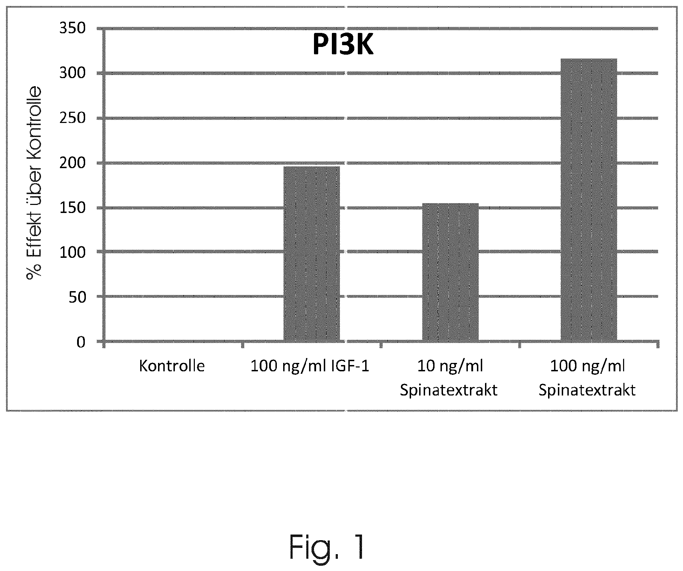

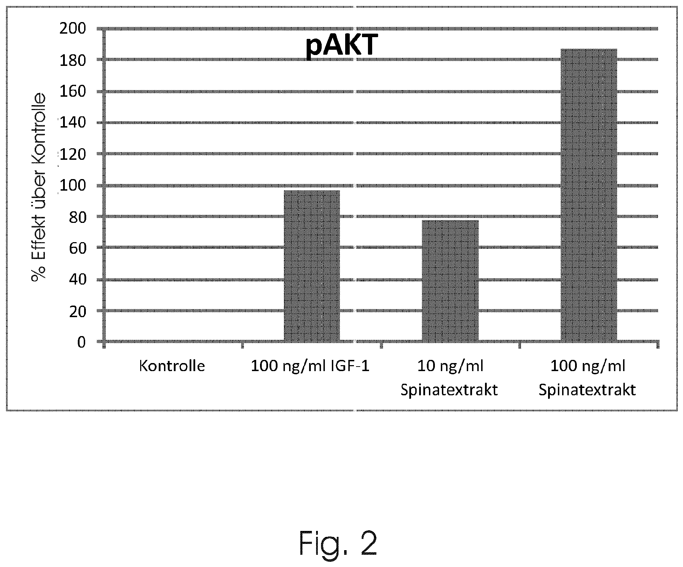

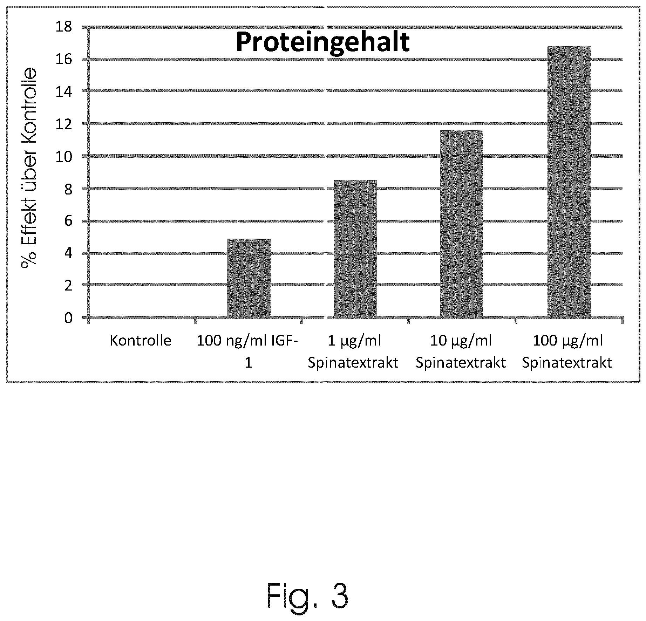

Die Resultate sind in den Figuren 1 bis 3 aufgeführt, dabei zeigen die Figuren 1 und 2 die Aktivierung des PI3K bzw. pAKT, die Figur 3 den Zuwachs an Proteingehalt der Zellen. Man sieht, dass sowohl schon eine 10 µg/ml, verstärkt aber eine 100 µg/ml Lösung effektive Aktivatoren, vergleichbar oder sogar übertreffend des IGF-1 darstellen. Eine erhöhte Proteinkonzentration in den Zellen, die eine Aktivierung durch IGF-1 übertrifft, ist sogar schon bei einer 1 µg/ml -Lösung nachweisbar.

Die einzelnen Kombinationen der Bestandteile und der Merkmale von den bereits erwähnten Ausführungen sind exemplarisch; der Austausch und die Substitution dieser Lehren mit anderen Lehren, die in dieser Druckschrift enthalten sind mit den zitierten Druckschriften werden ebenfalls ausdrücklich erwogen. Der Fachmann erkennt, dass Variationen, Modifikationen und andere Ausführungen, die hier beschrieben werden, ebenfalls auftreten können ohne von dem Erfindungsgedanken und dem Umfang der Erfindung abzuweichen. Entsprechend ist die obengenannte Beschreibung beispielhaft und nicht als beschränkend anzusehen. Das in den Ansprüchen verwendete Wort "umfassen" schließt nicht andere Bestandteile oder Schritte aus. Der unbestimmte Artikel "ein" schließt nicht die Bedeutung eines Plurals aus. Die bloße Tatsache, dass bestimmte Maße in gegenseitig verschiedenen Ansprüchen rezitiert werden, verdeutlicht nicht, dass eine Kombination von diesen Maßen nicht zum Vorteil benutzt werde kann. Der Umfang der Erfindung ist in den folgenden Ansprüchen definiert und den dazugehörigen Äquivalenten.

### Materialien und Methoden

Der Gehalt an β-Ecdyson kann mittels HPLC gemäß Serra et al., Brazilian Journal of Pharmacognosy 22(6): 1349-1354, Nov./Dec. 2012 gemessen werden.

Der Gehalt an Polyphenolen und Flavonoiden kann gemäß Everette et al, J. Agric. Food Chem. 2010, 58, 8139-8144 gemessen werden.

Die Absorption im UV/VIS kann wie folgend beschrieben gemessen werden, dabei handelt es sich um eine interne Methode zur Bestimmung der Farbwerte bei unterschiedlichen Wellenlängen aus verschiedenartigen Matrices:
Die entsprechenden Proben werden exakt eingewogen und in bidest. Wasser gelöst (Konzentration = 10 mg/mL; entspricht 1 %), wobei die Einwaage-Gefäße gelinde geschüttelt werden. Nach vollständigem Lösen der Proben (Sichtkontrolle) werden die Proben jeweils auf eine Konzentration von 0,5% und 0,1 % mit bidest. Wasser verdünnt (je nach Farbintensität). Die Proben werden in eine klare 96-Well-Platte verbracht, wobei die äußeren Wells nicht verwendet werden. In jedes Well wird exakt 250 µL pipettiert, wobei alle Proben sowie Blindwert dreifach bestimmt werden (also je 3 Wells). Alle Proben werden in Doppelbestimmung gemessen, d.h. es wurde eine doppelte Einwaage und Probenverdünnung vorgenommen. Die 96-Well Platten werden nach Verbringen in den Reader kurz von diesem geschwenkt, so dass eventuell vorhandene Luftbläschen die Bestimmung nicht beeinflussen kann. Die Messung erfolgt mittels eines Multi-Mode Microplate Reader der Serie Synergy 4 von Biotek, wobei die verwendete Software zur Steuerung Gen5 gewählt wird. Die Blindwerte und die Proben werden für die Farbbestimmung bei folgenden Wellenlägen gemessen: 440nm, 662 nm, 580 nm und 507 nm. Die Blindwerte (in diesem Fall bidest. Wasser) werden von den Probenwerten abgezogen. Die Temperatur betrug bei den beschriebenen Experimenten 23°C und wurde vom Gerät konstant gehalten. Die Auswertung erfolgte anhand der verwendeten Software.

Die antioxidative Wirkung (ORAC-Wert) kann gemäß Prior et al, J. Agric. Food Chem. 2005, 53, 4290-4302 gemessen werden.

## Patentansprüche

1. Spinatextrakt, enthaltend ≥ 1 % bis ≤ 10 % β-Ecdyson sowie ≥ 2 % bis ≤ 50 % Polyphenole, jeweils als Massenanteil im Trockenprodukt.

2. Extrakt gemäß Anspruch 1, wobei die Polyphenole Flavonoide enthalten.

3. Extrakt gemäß Anspruch 1 oder 2, wobei der Extrakt mindestens ein Flavonoid enthält, ausgewählt aus der Gruppe, umfassend:
5,3',4'-Trihydroxy-3-methoxy-6,7-methylenedioxyflavon-4'-O-glucuronid mit folgender Struktur: 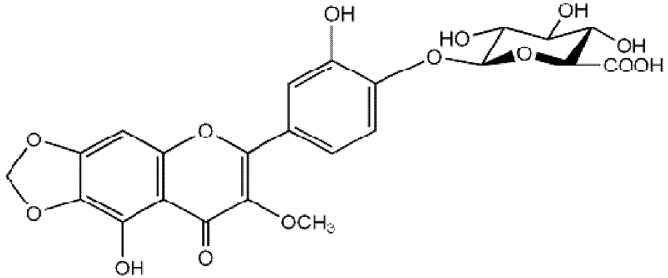
Jaceidin 4'-glucuronid = (3S,6S)-6-[4-(5,7-Dihydroxy-3,6-dimethoxy-4-oxochromen-2-yl)-2-methoxyphenoxy]-3,4,5-trihydroxyoxan-2-Carbonsäure mit folgender Struktur: 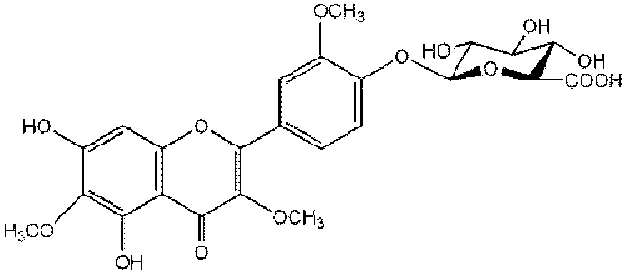
Spinacetin 3-gentiobiosid = 5,7-Dihydroxy-2-(4-hydroxy-3-methoxyphenyl)-6-methoxy-3-[(2R,5S)-3,4,5-trihydroxy-6-[[(2R,4S,5S)-3,4,5-trihydroxy-6-(hydroxymethyl)oxan-2-yl]oxymethyl]oxan-2-yl]oxychromen-4-on mit folgender Struktur: 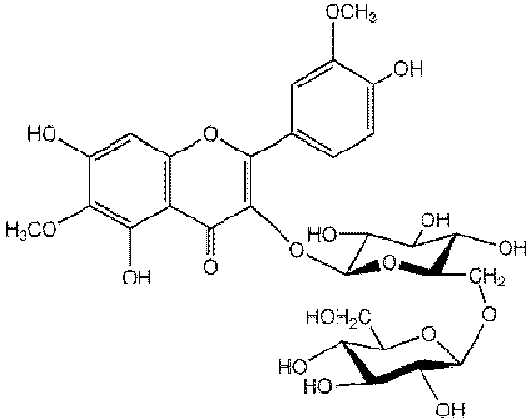
Isorhamnetin 3-sophoroside-7-glucosid = 3-[(2S,5S)-4,5-Dihydroxy-6-(hydroxymethyl)-3-[(2S,3R,5S)-3,4,5-trihydroxy-6-(hydroxymethyl)oxan-2-yl]oxyoxan-2-yl]oxy-5-hydroxy-2-(4-hydroxy-3-methoxyphenyl)-7-[(2S,4S,5S)-3,4,5-trihydroxy-6-(hydroxymethyl)oxan-2-yl]oxychromen-4-on mit folgender Struktur: 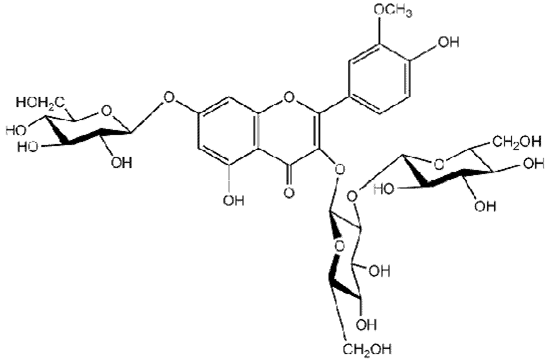
Spinatosid = (3,4',5,7-Tetrahydroxy-3',6-dimethoxyflavon mit folgender Struktur

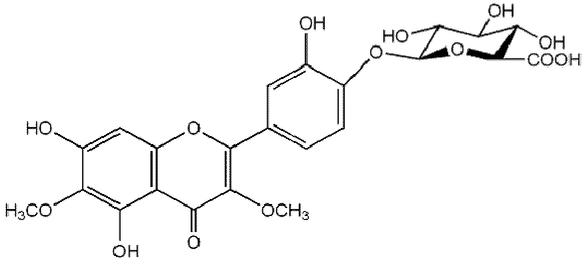

4. Extrakt gemäß einem der Ansprüche 1 bis 3, wobei der Extrakt Spinatosid zu 5,3',4'-Trihydroxy-3-methoxy-6,7-methylendioxy-flavon-4'-O-glucuronid im Verhältnis zwischen ≥ 1 : 0,1 und ≤ 1 : 20 enthält.

5. Verfahren zur Herstellung eines Spinatextrakts nach Anspruch 1, umfassend die Schritte:
a) Vorbehandeln des Spinats durch Blanchieren des Spinats mit Wasser oder einer wässrigen Lösung bei einer Temperatur von ≥ 70 °C für eine Zeitdauer von ≤ 10 Minuten, unter Erhalt eines Blanchierwassers;
b) Durchführung einer Adsorption des Blanchierwassers an einem Adsorberharz, worin das Adsorberharz ausgewählt ist aus der Gruppe, die besteht aus Copolymerisaten aus Ethylvinylbenzol und Divinylbenzol, Vinylpyrrolidon und Divinylbenzol, Vinylpyridin und Divinylbenzol, Styrol und Divinylbenzol, Polyaromaten, Polystyrol, Poly(meth)aycrylaten, Polypropylen, Polyestern, Polytetraflourethylen und Mischungen dieser Verbindungen.;
c) Elution mittels eines Lösemittels mit einem E_{T}30-Wert von ≥ 30 oder eines Lösemittelgemisches, enthaltend mindestens ein Lösemittel mit einem E_{T}30-Wert von ≥ 30; und
d) Aufkonzentrierung;

6. Verfahren nach Anspruch 5, wobei Schritt c) mittels eines Lösemittels mit einem E_{T}30-Wert von ≥ 45 oder einem Lösemittelgemisch, enthaltend mindestens ein Lösemittel mit einem E_{T}30-Wert von ≥ 45 durchgeführt wird.

7. Aromamischung, umfassend einen Spinatextrakt gemäß einem der Ansprüche 1 bis 4 in einer Menge von 0,1 bis 5 Gew.-%.

8. Nahrungsmittel und/oder Nahrungsergänzungsmittel, umfassend einen Spinatextrakt gemäß einem der Ansprüche 1 bis 4 oder eine Aromamischung gemäß Anspruch 7.

9. Tierfuttermittel, umfassend einen Spinatextrakt gemäß einem der Ansprüche 1 bis 4 in einer Menge von 0,1 bis 5 Gew.-% oder eine Aromamischung gemäß Anspruch 8.

10. Arzneimittel, umfassend einen Spinatextrakt gemäß der Ansprüche 1 bis 4 .

11. Arzneimittel zur Verwendung bei der Behandlung von Muskelatrophie, Muskelhypotonie und/oder Sarkopenie, umfassend einen Spinatextrakt gemäß der Ansprüche 1 bis 4.

12. Kosmetisches Mittel, umfassend einen Spinatextrakt gemäß einem der Ansprüche 1 bis 4.

13. Spinatextrakt gemäß einem der Ansprüche 1 bis 4 zur Verwendung als Arzneimittel.

## Claims

1. Spinach extract containing ≥ 1% to ≤ 10% β-ecdysone and ≥ 2% to ≤ 50% polyphenols, each as a percentage by weight in the dry product.

2. Extract according to claim 1, wherein the polyphenols contain flavonoids.

3. Extract according to claim 1 or 2, wherein the extract contains at least one flavonoid selected from the group comprising:
5,3',4'-trihydroxy-3-methoxy-6,7-methylenedioxyflavone-4'-O-glucuronide with the following structure: 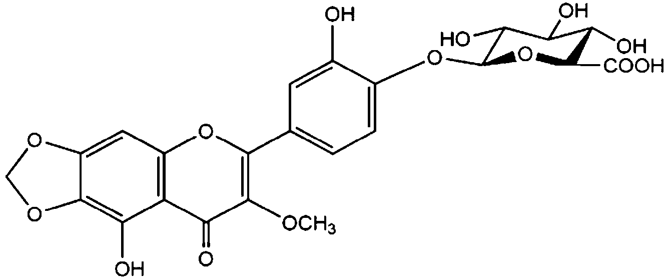
Jaceidin 4'-glucuronid = (3S,6S)-6-[4-(5,7-dihydroxy-3,6-dimethoxy-4-oxochromen-2-yl)-2-methoxyphenoxy]-3,4,5-trihydroxyoxane-2-carboxylic acid with the following structure: 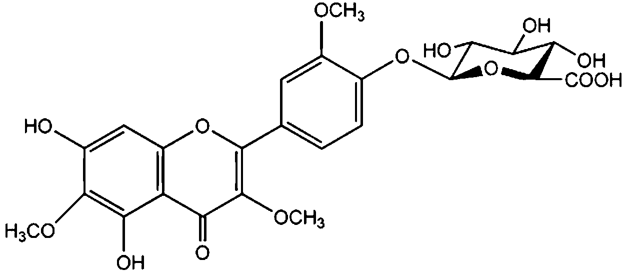
Spinacetin 3-gentiobiosid = 5,7-Dihydroxy-2-(4-hydroxy-3-methoxyphenyl)-6-methoxy-3- [(2R,5S)-3,4,5-trihydroxy-6-[[(2R,4S,5S)-3,4,5-trihydroxy-6-(hydroxymethyl)oxan-2-yl]oxymethyl]oxan-2-yl]oxychromen-4-one with the following structure: 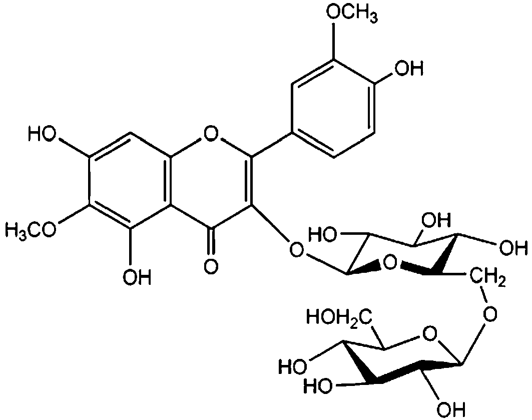
Isorhamnetin 3-sophoroside-7-glucosid = 3-[(2S,5S)-4,5-dihydroxy-6-(hydroxymethyl)-3-[(2S,3R,5S)-3,4,5-trihydroxy-6-(hydroxymethyl)oxan-2-yl]oxyoxan-2-yl]oxy-5-hydroxy-2-(4-hydroxy-3-methoxyphenyl)-7-[(2S,4S,5S)-3,4,5-trihydroxy-6-(hydroxymethyl)oxan-2-yl]oxychromen-4-one with the following structure: 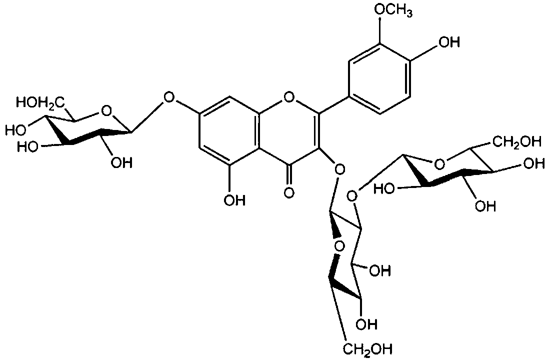
Spinatosid = (3,4',5,7-tetrahydroxy-3',6-dimethoxyflavone with the following structure:

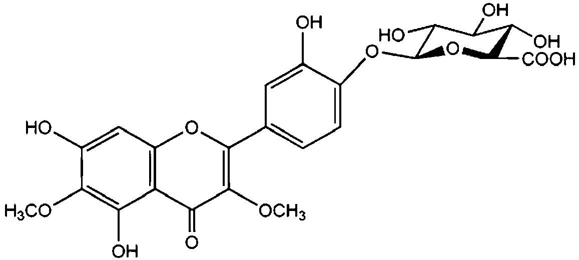

4. Extract according to any one of claims 1 to 3, wherein the extract contains spinatoside to 5,3',4'-trihydroxy-3-methoxy-6,7-methylenedioxyflavone-4'-O-glucuronide in a ratio of between ≥ 1 : 0.1 and ≤ 1 : 20.

5. Method for producing a spinach extract according to claim 1, comprising the steps:
a) pretreating the spinach by blanching the spinach with water or an aqueous solution at a temperature of ≥ 70 °C for a period of ≤ 10 minutes, obtaining blanching water;
b) performing adsorption of the blanching water on an adsorbent resin, wherein the adsorbent resin is selected from the group consisting of copolymers of ethylvinylbenzene and divinylbenzene, vinylpyrrolidone and divinylbenzene, vinylpyridine and divinylbenzene, styrene and divinylbenzene, polyaromatics, polystyrene, poly(meth)acrylates, polypropylene, polyesters, polytetrafluoroethylene, and mixtures of these compounds;
c) Elution using a solvent with an E_{T}30 value of ≥ 30 or a solvent mixture containing at least one solvent with an E_{T}30 value of ≥ 30; and
d) Concentration;

6. Method according to claim 5, wherein step c) is carried out using a solvent with an E_{T}30 value of ≥ 45 or a solvent mixture containing at least one solvent with an E_{T}30 value of ≥ 45.

7. Aroma mixture comprising a spinach extract according to one of claims 1 to 4 in an amount of 0.1 to 5% by weight.

8. Food and/or dietary supplements comprising a spinach extract according to any one of claims 1 to 4 or a flavor mixture according to claim 7.

9. Animal feed comprising a spinach extract according to any one of claims 1 to 4 in an amount of 0.1 to 5% by weight or a flavor mixture according to claim 8.

10. Medicinal product comprising a spinach extract according to claims 1 to 4.

11. Medicinal product for use in the treatment of muscle atrophy, muscle hypotonia, and/or sarcopenia, comprising a spinach extract according to claims 1 to 4.

12. Cosmetic product comprising a spinach extract according to any one of claims 1 to 4.

13. Spinach extract according to any of claims 1 to 4 for use as a medicinal product.

## Revendications

1. Extrait de epinard contenant ≥ 1 % à ≤ 10 % de β-ecdysone ainsi que ≥ 2 % à ≤ 50 % de polyphénols, respectivement en pourcentage massique dans le produit sec.

2. Extrait selon la revendication 1, les polyphénols contenant des flavonoïdes.

3. Extrait selon la revendication 1 ou 2, dans lequel l'extrait contient au moins un flavonoïde choisi dans le groupe comprenant:
5,3',4'-trihydroxy-3-méthoxy-6,7-méthylènedioxyflavone-4'-O-glucuronide ayant la structure suivante: 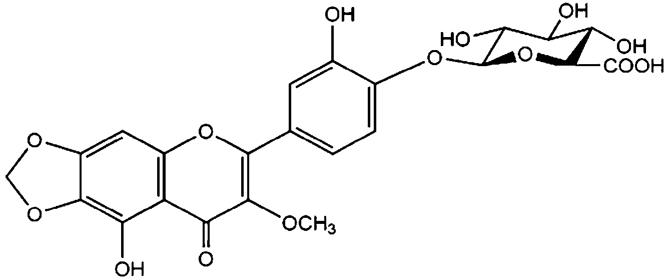
Jaceidin 4'-glucuronide = (3S,6S)-6-[4-(5,7-dihydroxy-3,6-diméthoxy-4-oxochromène-2-yl)-2-méthoxyphénoxy]-3,4,5-trihydroxyoxane-2-acidecarboxylique ayant la structure suivante: 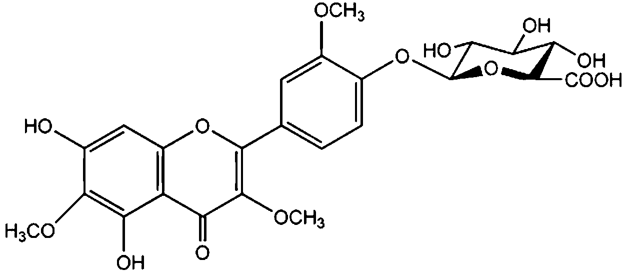
Spinacetin 3-gentiobioside = 5,7-dihydroxy-2-(4-hydroxy-3-méthoxyphényl)-6-méthoxy-3- [(2R,5S)-3,4,5-trihydroxy-6-[[(2R,4S,5S)-3,4,5-trihydroxy-6-(hydroxyméthyl)oxan-2-yl]oxyméthyl]oxan-2-yl]oxychromène-4-one ayant la structure suivante: 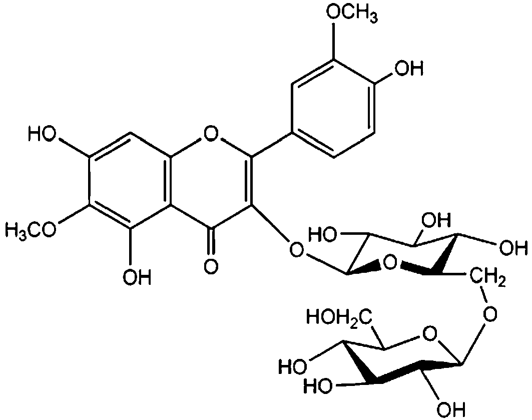
Isorhamnetin 3-sophoroside-7-glucoside = 3- [(2S,5S)-4,5-dihydroxy-6-(hydroxyméthyl)-3-[(2S,3R,5S)-3,4,5-trihydroxy-6-(hydroxyméthyl)oxan-2-yl]oxyoxan-2-yl]oxy-5-hydroxy-2-(4-hydroxy-3-méthoxyphényl)-7- [(2S,4S,5S)-3,4,5-trihydroxy-6-(hydroxyméthyl)oxan-2-yl]oxychromène-4-one ayant la structure suivante: 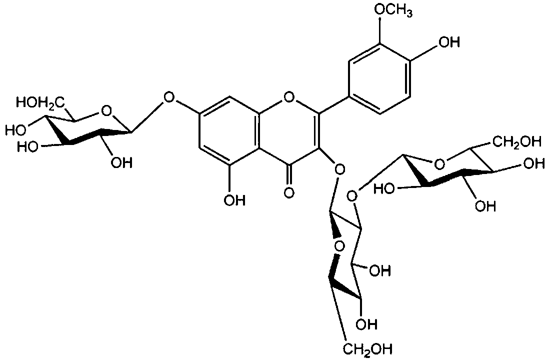
Spinatosid = 3,4',5,7-tétrahydroxy-3',6-diméthoxyflavone ayant la structure suivante:

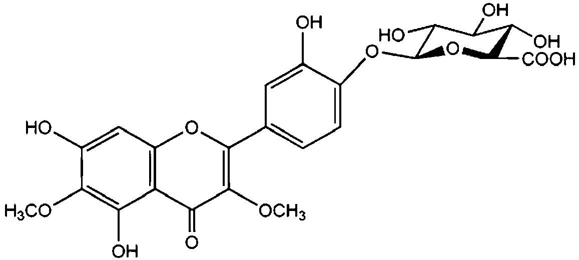

4. Extrait selon l'une quelconque des revendications 1 à 3, dans lequel l'extrait contient du spinatoside en 5,3',4'-trihydroxy-3-méthoxy-6,7-méthylènedioxy-flavone-4'-O-glucuronide dans un rapport compris entre ≥ 1 : 0,1 et ≤ 1 : 20.

5. Procédé de fabrication d'un extrait d'épinards selon la revendication 1, comprenant les étapes suivantes:
a) prétraitement des épinards par blanchiment à l'eau ou dans une solution aqueuse à une température ≥ 70 °C pendant une durée ≤ 10 minutes, afin d'obtenir une eau de blanchiment ;
b) réalisation d'une adsorption de l'eau de blanchiment sur une résine adsorbante, dans laquelle la résine adsorbante est choisie dans le groupe constitué par les copolymères d'éthylvinylbenzène et de divinylbenzène, de vinylpyrrolidone et de divinylbenzène, de vinylpyridine et de divinylbenzène, de styrène et de divinylbenzène, de polyaromatiques, de polystyrène, de poly(méth)acrylates, de polypropylène, de polyesters, de polytétrafluoroéthylène et de mélanges de ces composés;
c) Éluation à l'aide d'un solvant ayant une valeur E_{T}30 ≥ 30 ou d'un mélange de solvants contenant au moins un solvant ayant une valeur E_{T}30 ≥ 30; et
d) Concentration;

6. Procédé selon la revendication 5, dans lequel l'étape c) est réalisée à l'aide d'un solvant ayant une valeur E_{T}30 ≥ 45 ou d'un mélange de solvants contenant au moins un solvant ayant une valeur E_{T}30 ≥ 45.

7. Mélange aromatique comprenant un extrait d'épinards selon l'une des revendications 1 à 4 en une quantité de 0,1 à 5 % en poids.

8. Aliment et/ou complément alimentaire comprenant un extrait d'épinards selon l'une des revendications 1 à 4 ou un mélange aromatique selon la revendication 7.

9. Aliment pour animaux comprenant un extrait d'épinards selon l'une des revendications 1 à 4 en une quantité de 0,1 à 5 % en poids ou un mélange aromatique selon la revendication 8.

10. Médicament comprenant un extrait d'épinard selon les revendications 1 à 4.

11. Médicament destiné à être utilisé dans le traitement de l'atrophie musculaire, de l'hypotonie musculaire et/ou de la sarcopénie, comprenant un extrait d'épinards selon les revendications 1 à 4.

12. Produit cosmétique comprenant un extrait d'épinard selon l'une des revendications 1 à 4.

13. Extrait d'épinard selon l'une des revendications 1 à 4, destiné à être utilisé comme médicament.
